# EUROPEAN PATENT SPECIFICATION

(11) **EP 1 049 478 B1**
(45) Date of publication and mention of the grant of the patent: **04.09.2002**
(21) Application number: 99900405.4
(22) Date of filing: 13.01.1999
(51) Int. Cl.: A61K 31/13, A61P 35/00, A61P 43/00

(54) **COMPOSITION CONTAINING PROPARGYLAMINE FOR ENHANCING CANCER THERAPY**
PROPARGYLAMIN ENTHALTENDE ZUSAMMENSETZUNG ZUR VERBESSERUNG DER KREBSTHERAPIE
METHODE PERMETTANT D'AMELIORER LE TRAITEMENT DU CANCER

(30) Priority: 13.01.1998 US 71023 P
(43) Date of publication of application: 08.11.2000
(73) Proprietor: University of Saskatchewan Technologies Inc., Saskatoon, Saskatchewan S7N 5C8 (CA)
(72) Inventor: PATERSON, I., Alick, deceased (CA); WARRINGTON,R.C., Univ Saskatchewan, Neurpsy. Res., Saskatoon, Saskatchewan S7N 5E4 (CA); BOULTON, Alan, A., Saskatoon, Saskatchewan S7K 0C9 (CA)
(74) Representative: Couchman, Jonathan Hugh
(86) International application number: CA9900005
(87) International publication number: WO99036076

(56) References cited:
- US-A- 4 460 599
- US-A- 5 169 868

## Description

The present invention relates to the use of propargylamines of formula I below for the preparation of a medicament for enhancing cancer therapy by administering an effective amount of an antineoplastic modulator. Preferred antineoplastic modulators are propargylamines including aliphatic propargylamines and aromatic propargylamines. The invention also includes a pharmaceutical composition for enhancing the treatment of cancer comprising an effective amount of an antineoplastic modulator of the present invention in admixture with a suitable diluent or carrier.

### BACKGROUND OF THE INVENTION

Cancer is a collection of diseases involving inappropriate and unregulated growth of cells in the body. The aim of chemical therapy (chemotherapy) of cancer is to introduce a chemical (antineoplastic drug) which will kill the cancerous cells but will not damage normal cells. The early rationale for the development of conventional antineoplastic drugs was that such agents would act selectively on cells undergoing cell division; since cancerous cells were thought to be invariably dividing more rapidly than normal cells in the body, it was believed that this would offer some therapeutic selectivity. However, antineoplastic agents collectively have the lowest therapeutic indices of any class of drugs used in humans. This lack of selectivity leads to the severe side effects associated with cancer chemotherapy; the major dose-limiting consideration for use of these agents is toxicity to bone marrow. Furthermore, the poor selectivity of these agents means they must be used at sub-optimal doses. The latter, in turn, may cause the development of a variety of drug resistance traits by cancerous cells. Thus, many types of cancers are ultimately unresponsive to chemotherapy and are therefore incurable.

Notwithstanding such limitations, chemotherapy remains the only and thus the most important treatment option for disseminated cancers. Despite decades of effort to find more effective and less toxic agents, the poor response of patients to conventional anticancer drugs and the limitations arising from intrinsic or acquired drug-resistance continue to limit the chemotherapeutic approach. It is estimated that over 50% of patients with advanced cancer will fail to respond, or will relapse from their initial response to chemotherapy, and will thus ultimately succumb to their disease. Given the prevalence and severity of disseminated disease, improving the chemotherapeutic treatment modality nevertheless remains a crucial objective of cancer research (1).

One novel and potentially major means of improving the chemotherapeutic modality of cancer treatment would be to improve the selectivity of the currently-available agents. To the degree to which selectivity could be improved, such an approach would diminish the toxic side effects and allow treatment with more appropriate doses of antineoplastics which, in turn, would diminish the inadvertent selection of drug-resistance variants during treatment. If, in addition, such a strategy would circumvent drug-resistance traits of either the intrinsic or acquired types, it would diminish all of the major, known limitations to conventional cancer chemotherapy. Remarkably, such an approach has been developed and verified to have all of these advantages in experimental chemotherapeutic models (2- 17). Termed the modulator approach for improving cancer chemotherapy, this novel strategy solves the major limitations otherwise associated with the use of conventional antineoplastics.

An antineoplastic modulator is a chemical which modifies the action of an antineoplastic drug, improving the selectivity, and therefore efficacy of the antineoplastic drug. An antineoplastic modulator acts, simultaneously, to advantage in three ways: i), it protects non-cancerous (normal) tissue from the toxic effects of the antineoplastic drug; ii), it increases the ability of the antineoplastic drug to kill cancerous cells, and iii), it suppresses the drug resistance traits exhibited by many cancerous cells.

The present inventors have prepared many novel propargylamines as described in United States Patent No. 5,169,868 and 5,840,979. The inventors have shown that the novel propargylamines are useful as MAO-B inhibitors and are useful in treating various neuropsychiatric disorders including Parkinson's disease, Alzheimer's disease, depression, attention deficit disorder, hyperactive disorders as well as other aging-associated diseases.

Surprisingly, the present inventors have found that the propargylamines are also useful as antineoplastic modulators and can enhance the effect of antineoplastic drugs.

### SUMMARY OF THE INVENTION

The present inventors have shown that propargylamines enhance the killing of tumor cells by antineoplastic drugs and protect normal cells from the cytotoxic effects of antineoplastic drugs. Consequently, propargylamines are well-suited to enhance any chemotherapy regime and can increase the effectiveness while reducing the side-effects of cancer therapy.

The present invention provides a use of a propargylamine of formula I for the preparation of a medicament to be used in the therapeutic methods described herein.

Specifically, the medicaments are for (i) enhancing the activity of an antineoplastic drug, (ii)increasing the sensitivity of a tumor to an antineoplastic drug and/or (iii) protecting normal cells from the cytotoxic effects of an antineoplastic drug.

The present invention also includes a pharmaceutical composition useful for treating cancer comprising an antineoplastic drug and an effective amount of a propargylamine of formula I.

Other features and advantages of the present invention will become apparent from the following detailed description.

### BRIEF DESCRIPTION OF THE DRAWINGS

The invention will now be described in relation to the drawings in which:
Figure 1 is graph showing the RATIO of various antineoplastic modulators versus the concentration of the antineoplastic modulator. The definition of RATIO is provided in Example 1.
Figure 2 is a graph showing the relative cell survival of normal bone marrow versus time, in the presence of various modulators. HISOL=histindinol, cis=cisplatinum, 2HPA=R-2HPA.
Figure 3 is a graph showing the relative cell survival of cancer cells versus time, in the presence of various modulators.

### DETAILED DESCRIPTION OF THE INVENTION

The present inventors have shown that propargylamines of formula I below enhance the killing of tumor cells by antineoplastic drugs and protect normal cells from the cytotoxic effects of antineoplastic drugs. In addition, the propargylamines have been shown to overcome a drug-resistance attribute of tumor cells. *In vivo* data is included which verifies that these three powerful attributes of the approach are operative in live, tumor bearing animals. Consequently, propargylamines are well-suited to enhance any chemotherapy regime.

### Propargylamines

The propargylamines that may be included in the compositions of the present invention are of the general 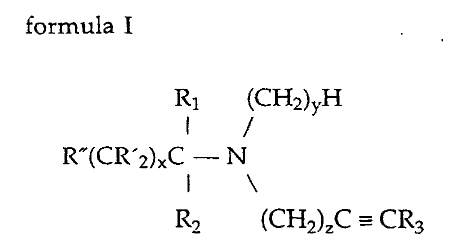 wherein
x is an integer ranging from 0 to 13;
y is an integer ranging from 0 to 5;
z is 1;
R₁, R₂ and R₃ are the same or different and represent hydrogen or a straight chain or branched lower alkyl; and
R' and R" are the same or different and represent hydrogen, phenyl or a halogen and pharmaceutically acceptable salts thereof.

The lower alkyl has between 1 and 4 carbon atoms and the halogen atom is selected from fluorine, chlorine, bromine and iodine. More preferably, the lower alkyl is selected from methyl.

In another embodiment, the propargylamine is of the general formula I wherein y is 1 and the pharmaceutically acceptable salts thereof. A preferred propargylamine of the formula I wherein y is 1 is R-2-heptylmethylpropargylamine (R-2HMP).

Other propargylamines of the formula I wherein y is 1 include:
N-(1-Propyl) N-methylpropargylamine;
N-(2-Propyl) N-methylpropargylamine;
N-(1-Butyl) N-methylpropargylamine;
N-(1-Pentyl) N-methylpropargylamine;
N-(1-Hexyl) N-methylpropargylamine;
N-(1-Heptyl) N-methylpropargylamine;
N-(1-Octyl) N-methylpropargylamine;
N-(1-Nonyl) N-methylpropargylamine;
N-(1-Decyl) N-methylpropargylamine;
N-(1-Undecyl) N-methylpropargylamine;
N-(1-Dodecyl) N-methylpropargylamine;
(R)-N-(2-Butyl) N-methylpropargylamine;
(R)-N-(2-Pentyl) N-methylpropargylamine;
(R)-N-(2-Hexyl) N-methylpropargylamine;
(R)-N-(2-Heptyl) N-methylpropargylamine;
(R)-N-(2-Octyl) N-methylpropargylamine;
(R)-N-(2-Octyl) N-methylpropargylamine;
(R)-N-(2-Decyl) N-methylpropargylamine;
(R)-N-(2-Undecyl) N-methylpropargylamine; and
(R)-N-(2-Dodecyl) N-methylpropargylamine.

In yet another embodiment, the propargylamine is of the general formula I, described above, wherein y is 0, and the pharmaceutically acceptable salts thereof. A preferred propargylamine of the formula I where y=0, is R-2-heptyl-propargylamine (R-2HPA).

Other compounds of the formula I, wherein y is 0, include:
N-(1-Propyl) propargylamine;
N-(2-Propyl) propargylamine;
N-(1-Butyl) propargylamine;
N-(1-Pentyl) propargylamine;
N-(1-Hexyl) propargylamine;
N-(1-Heptyl) propargylamine;
N-(1-Octyl) propargylamine;
N-(1-Nonyl) propargylamine;
N-(1-Decyl) propargylamine;
N-(1-Undecyl) propargylamine;
N-(1-Dodecyl) propargylamine;
(R)-N-(2-Butyl) propargylamine;
(R)-N-(2-Pentyl) propargylamine;
(R)-N-(2-Hexyl) propargylamine;
(R)-N-(2-Heptyl) propargylamine;
(R)-N-(2-Octyl) propargylamine;
(R)-N-(2-Octyl) propargylamine;
(R)-N-(2-Decyl) propargylamine;
(R)-N-(2-Undecyl) propargylamine; and
(R)-N-(2-Dodecyl) propargylamine.

The preferred propargylamines of the chiral compounds of the formula I are the R-enantiomers.

In a further embodiment, the propargylamine is R-deprenyl. R-deprenyl is a compound of the formula I wherein R₁ is methyl, R₂ is hydrogen, R" is phenyl, R' is hydrogen, x is 1, y is 1, z is 1 and R₃ is hydrogen.

In another embodiment, the propargylamine is R-desmethyldeprenyl. R-desmethyldeprenyl is a compound of the formula I wherein R₁ is methyl, R₂ is hydrogen, R" is phenyl, R' is hydrogen, x is 1, y is 0, z is 1 and R₃ is hydrogen.

In yet another embodiment, the propargylamine is Rasagiline having the following formula II:

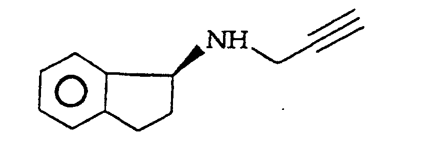

All of the above described propargylamines may be collectively referred to as "the propargylamines of the invention".

The propargylamines of the present invention may be prepared using techniques known in the art. For example, the aliphatic propargylamines may be prepared as described in the inventors United States Patent No. 5,169,868 and 5,840,979. Briefly, the compounds may be prepared by condensing propargyl bromide with a chiral aliphatic amine or N-methylamine in the presence of a base and recovering the desired compound. Preferably the R-enantiomers are prepared.

### Therapeutic Uses

The invention provides a use of a propargylamine of formula I for the manufacture of a medicament to enhance the effect of an antineoplastic drug.

The term "effective amount" as used herein means an amount effective, at dosages and for periods of time necessary to achieve the desired result.

The term "animal" as used herein means any member of the animal kingdom including all mammals, birds, fish, reptiles and amphibians. Preferably, the animal to be treated is a mammal, more preferably a human.

One method by which the propargylamines of the invention may enhance the effect of an antineoplastic drug is by increasing the sensitivity of the tumor to the drug. Accordingly, in one aspect, the present invention relates to use of a propargylamine of formula I for the manufacture of a medicament to increase the sensitivity of a tumor to an antineoplastic agent. The tumor may be one that is resistant to cancer therapy such as a multidrug resistant tumor or a radioresistant tumor.

Another method by which the propargylamines of the invention may enhance the effect of an antineoplastic drug is by protecting normal cells from the cytotoxic effects of the drug. Accordingly, in another aspect, the present invention provides a use of a propargylamine of formula I for the manufacture of a medicament to protect normal cells from the cytotoxic effects of an antineoplastic drug.

Further aspects of the invention are set forth in the claims.

The propargylamines of the invention can be used to enhance the treatment of all forms of cancer or malignant diseases for which chemotherapy is a bona fide treatment option. These malignancies include, but are not limited to, leukemias, lymphomas (Hodgkins and non-Hodgkins), plasmacytomas, histiocytomas, melanomas, adenomas, sarcomas, carcinomas of solid tissues, hypoxic tumours, squamous cell carcinomas, genitourinary cancers such as cervical and bladder cancer, hematopoietic cancers, head and neck cancers, and nervous system cancers. Treatment with the propargylamine modulators may allow for treatment of tumors that are resistant to chemotherapy. The latter are diverse, but one common, well-studied example is the so-called multi-drug resistant (MDR) tumor cells. MDR tumors include adenocarcinomas, neuroblastoma cells, leukemias, lymphomas, breast cancer and ovarian cancer cells. Treatment with the propargylamine modulators may also allow for more effective radiotherapy of tumours that currently respond poorly to radiotherapy such as adenocarcinomas of the bowel and lung.

Antineoplastic drugs which may be potentiated or enhanced by the propargylamine modulators can be any antineoplastic drug including known, conventional drugs as well as those yet to be identified. Examples of classes of antineoplastic agents include antimetabolites, alkylating agents, antimicrobial antineoplastics, antimicrotubule agents, cisplatinum and its derivatives and the topoisomerase interactive agents. In particular, chemotherapeutic agents amenable to this modulatory effect may include but are not limited to, adriamycin, BCNU and CCNU (i.e., bis (2-chloroethyl)-3-cyclohexyl-1-nitrosurea and 1-(2-chloroethyl)-3-cyclohexyl -1-nitrosourea, respectively, bleomycin sulfate, camptothecin, carmustine, chlorambucil, cisplatinum, cyclophospamide, cytosine arabinoside, daunomycin/daunorubicin, dacarbazine, doxorubicin, 5-fluorouracil, melphalan, mitomycin, mitoxantrone hydrochloride, etoposide, streptozocin and taxol and taxol derivatives.

Although the propargylamines of the invention may be administered before, after and/or concurrently with the antineoplastic drug, they are likely best administered prior to chemotherapy.

### Pharmaceutical Compositions

The propargylamines of the invention may be incorporated into a pharmaceutical composition along with an antineoplastic drug which may be useful in enhancing the activity of an antineoplastic drug, increasing the sensitivity of a tumor to an antineoplastic drug and/or protecting normal cells from the cytotoxic effects of an antineoplastic drug. The pharmaceutical compositions may be useful for treating cancer.

The pharmaceutical compositions of the invention can be prepared by per se known methods for the preparation of pharmaceutically acceptable compositions which can be administered to patients, and such that an effective quantity of the active substance is combined in a mixture with a pharmaceutically acceptable vehicle. Suitable vehicles are described, for example, in Remington's Pharmaceutical Sciences (Remington's Pharmaceutical Sciences, Mack Publishing Company, Easton, Pa., USA 1985). The pharmaceutical compositions of the invention can be for oral, topical, rectal, parenteral, local, intravenous, inhalant or intracerebral use. They may be in solid or semisolid form, for example pills, tablets, creams, gelatin capsules, capsules, suppositories, soft gelatin capsules, gels, membranes, tubelets. For parenteral and intracerebral uses, those forms for intramuscular or subcutaneous administration can be used, or forms for infusion or intravenous or intracerebral injection can be used, and can therefore be prepared as solutions of the active compounds or as powders of the active compounds to be mixed with one or more pharmaceutically acceptable excipients or diluents, suitable for the aforesaid uses and with an osmolarity which is compatible with the physiological fluids. For local use, those preparations in the form of creams or ointments for topical use or in the form of sprays should be considered; for inhalant uses, preparations in the form of sprays, for example nose sprays, should be considered. Dosages to be administered depend on individual needs, on the desired effect and on the chosen route of administration, but daily dosages to humans by subcutaneous, intramuscular, intravenous or intracerebral injection generally vary between 100 ng and 100 µg of active substance per kg body weight, preferably between 1 µg and 50 µg per kg body weight for the aliphatic propargylamines. For aromatic propargylamines, the above doses may be increased ten fold.

The following examples are illustrative of the present invention:

### EXAMPLES

### EXAMPLE 1

### In Vitro Protocol for Assessing the Capacity of Various Compounds to Modulate Cisplatinum Toxicity

The protocol detailed below can be used for any normal/tumorigenic cell pair which will attach to plastic. Non-adherent lines (which includes most tumor cells, including NW16; consult references 5-10) require quantitation in soft agar. The present experiments are based on i) rat2 cells, a phenotypically normal, established rat fibroblast line and, ii) a tumorigenic derivative thereof, NW16 cells, which are rat2 cells transformed by a Fujinami sarcoma virus oncogene (see work of A. Pawson and P130^{gag-fps}). Rat2 and NW16 cells are maintained in a sub-confluent randomly-proliferating state in Dulbecco's modified minimal essential media with 10% (vol/vol) calf serum in plates incubated at 37°C in a humidified CO₂ (10%) incubator. All experiments reported rely on clonogenic cell survival assays (see references 4-6). Assays using rat2 (normal cells) were performed as follows: cells are exposed, in 10 cm culture dishes, to various drugs in media plus serum for varying lengths of time; seeding is at varying cell numbers, over log₁₀ ranges, depending upon the degree of killing anticipated. [For the figure presented, incubation was for 72 hours prior to washing and assessment of clonogenic survival]. Both control and the experimental cultures are then gently washed, twice, with phosphate buffered saline, then once more with media minus serum, and then left in media plus serum, undisturbed until macroscopic colonies appear (7-9 days of incubation). The colonies are then fixed and stained with saturated methylene blue in 50% methanol and counted. The number of colonies, evaluated from 2 or more sets of duplicate cultures seeded at initial densities differing by factors of 10, are determined and converted to relative number of colonies, using the 0-hour control value as 1.0. Assays of NW16 cells were similar; however, because these cells are poorly adherent, following drug exposure, the washing procedure is modified, as is the quantitation of survivors step. In the latter case, quantitation requires plating the cells in soft agar (references 5-10).

### Presentation of Results by "RATIO" Method

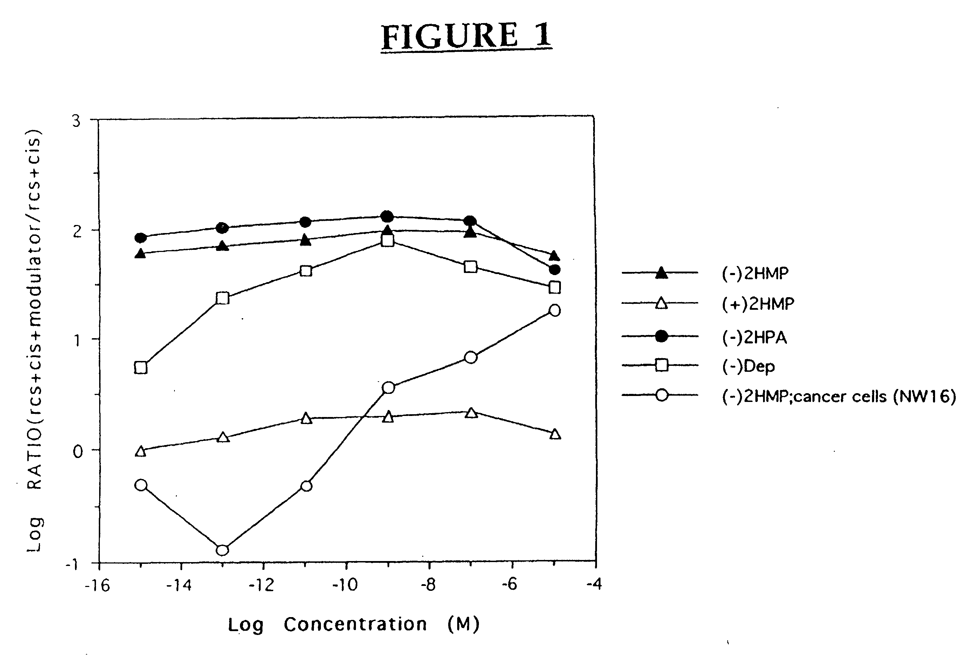

A simplified presentation of the data, by the RATIO method, is show in Figure 1. By dividing the relative cell survival (R.C.S.) value obtained in cultures which have been exposed to the combination of anticancer drug (in this case, cisplatinum) and modulator by the corresponding R.C.S. value obtained for the anticancer drug alone reveals both the nature and the magnitude of the effect mediated by the modulator. Ratios greater than unity indicate that the modulator has conferred a protective response, whereas ratios less than unity indicate an enhanced cell killing.

### Results

As can be seen from Figure 1, R-2-heptyl-propargylamine (R-2HPA), the desmethyl metabolite of R-2-heptyl-methyl propargylamine (R-2HMP), and R-2HMP (the pro-drug) are effective, over a wide concentration range (10⁷ - 10⁻¹⁵ M), at protecting normal fibroblasts which are p53 dependent. R-2HPA is the more potent. R-Deprenyl whilst active, is less efficacious over a more limited concentration range (10⁻⁷ - 10⁻¹³ M). The usually inactive pro-drug isomer S-2HMP is also inactive in this assay. In the tumorigenic cells (mutants in which p53 is absent) it can be seen that enhanced killing by cisplatinum occurs in the range (10⁻¹¹ - 10⁻¹⁵ M) but with a reversal to a protective effect when the concentration of R-2HMP is 10⁻⁹M or greater.

### Summary

R-2HMP and R-2HPA both protect normal cells and enhance the killing of tumor cells in the presence of cisplatinum in this *in vitro* fibroblast model. The protection and the enhanced killing occur in the 10⁻¹¹ - 10⁻¹⁵ M range. R-Deprenyl was also effective over a more limited concentration, in the 10⁻⁷ to 10⁻¹³ M range. Since L-histidinol exhibits similar properties (although higher doses are required) in this and several other *in vitro* and *in vivo* paradigms, and in the presence of other anticancer drugs, it is reasonable to predict that R-2HMP, R-2HPA and the other aliphatic propargylamines, by analogy, will also exhibit activity in these other systems.

### EXAMPLE 2

### In Vivo Assessment of Anticancer Drug Modulators: Effects of R-2HPA

Seven groups of mice were treated and assessed in this model as follows:
1. Nil control (1 mouse)
2. P388 control (1 mouse)
3. Cisplatinum (5 mice)
4. Histidinol (2 mice)
5. Histidinol + cisplatinum (5 mice)
6. R-2HPA (4 mice)
7. R-2HPA + cisplatinum (5 mice)

P388 cells (1 million) were injected into the tail vein of 22 female DBA/2J mice (Protocol first developed in reference 6 and 8). The mice were then randomly divided into the above groups and injected (ip) with drugs 96h later. Doses were cisplatinum 0.2 mg at 0 hour; Histidinol 5 mg/injection and R-2HPA 0.38 ug/injection; administered 5 times at -2, 0, +2, +4, and +6 hours. 48 h after drug treatment, cells from the femurs of the mice were harvested, washed and plated (at log10 dilutions) so as to allow quantitative and specific relative cell survival values to be generated for the responses of normal femoral bone marrow cells (specifically, CFU-C/GM or granulocyte/macrophage precursor cells) and clonogenic P388 leukemia cells (8).

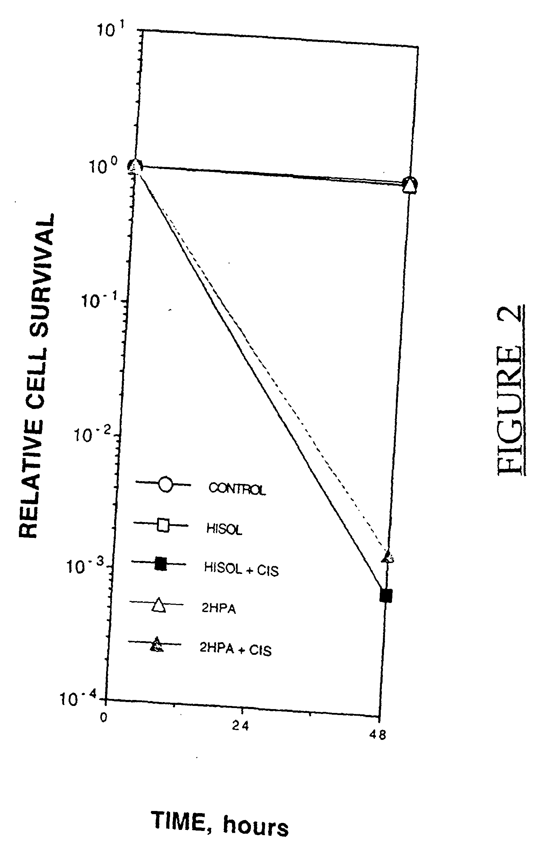

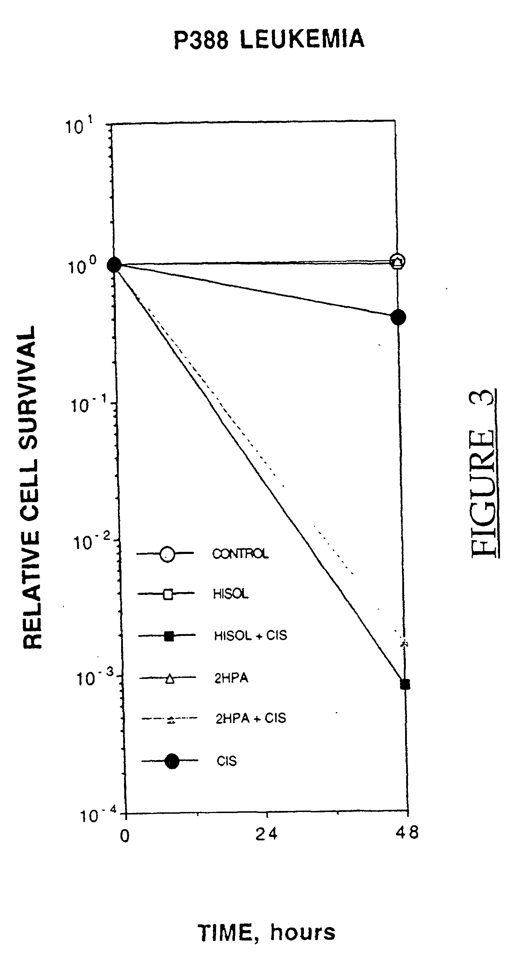

As can be seen in Figure 2, both histidinol and R-2HPA were effective at protecting normal bone marrow cells, whereas in Figure 3, it can be seen that both histidinol and R-2HPA enhanced the killing by cisplatinum of P388 cells. It should be emphasized that the P388 leukemic line is substantially resistant to the cisplatinum (relative to the responses of the CFU-c/GM cells). This is an example of the poor therapeutic index common to conventional antineoplastics. In this example, the cisplatinum, when used alone, can be seen to be about 100-times more effective at killing the crucial normal marrow cells than it is for killing the intrafemoral leukemia (tumor) cells. In the presence of the modulators histidinol and R-2HPA, the therapeutic index of cisplatinum is vastly improved; thus, the toxicity to the marrow cells is essentially eliminated and the toxicity to the leukemia cells is increased by almost a 1000-fold. In other words, both histidinol and R-2HPA are simultaneously protecting the most vulnerable normal cells from cancer drug toxicity and simultaneously circumventing a profound drug-resistance trait. That these effects are observed *in vivo* (i.e., in live animals) and in the same tissue of those animals cannot be over-emphasized in terms of its potential capacity to improve chemotherapy, in as much as it reveals clearly and dramatically the ability of modulators to improve selectivity, efficacy and to circumvent the problem of drug-resistance shown by tumor cells. It can also be seen that this remarkable effect is obtained with R-2HPA at the low dose of 0.38 ug, producing a therapeutic index of about 50,000 between the protection of healthy normal cells and the killing of the cancerous cells. This effect is known to be p53 dependent vis a vis histidinol and it is likely to be the same with R-2HPA.

The modulator strategy has been shown to be remarkably effective in many *in vivo* tumor models (4-6; 7-11), in numerous types of human cancer cells (12,13) and in many kinds of drug resistance traits (5; 16,17). Consequently, considering the data cited herein, it is predicted that the use of propargylamines as antineoplastic modulators will improve the chemotherapeutic management of a wide variety of human malignant disease types which will include non-resistant, intrinsic and acquired drug-resistance types. The modulator approach has been validated experimentally to markedly improve treatment of malignancies of myeloid origin (leukemias, lymphomas, and cancers of "blood cell" origin; (7-10) and for disseminated or metastatic disease (11); these are the situations wherein chemotherapy is often the only available clinical treatment option, the least responsive to treatment and/or the most prone to failure due to either intrinic or acquired drug-resistance and hence incurable status.

While the present invention has been described with reference to what are presently considered to be the preferred examples, it is to be understood that the invention is not limited to the disclosed examples.

## Claims

1. A use of a propargylamine for the manufacture of a medicament to enhance the activity of an antineoplastic drug wherein the propargylamine is of the general formula I 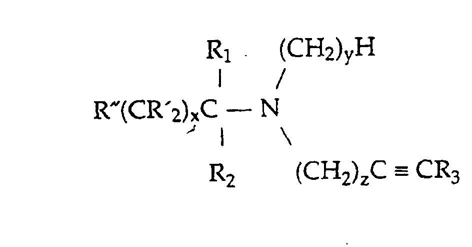 wherein
x is an integer ranging from 0 to 13;
y is an integer ranging from 0 to 5;
z is 1;
R₁, R₂ and R₃ are the same or different and represent hydrogen or a straight chain or branched C₁₋₄ alkyl; and
R' and R" are the same or different and represent hydrogen, phenyl or a halogen and pharmaceutically acceptable salts thereof.

2. A use according to claim 1 wherein the medicament is for increasing the sensitivity of a tumor to the antineoplastic drug.

3. A use according to claim 2 wherein the tumor is a drug resistant tumor.

4. A use according to claim 1 wherein the medicament is for protecting normal cells from the cytotoxic effects of the antineoplastic drug.

5. A use of (a) a propargylamine of the formula I as defined in claim 1 and (b) an antineoplastic drug for the manufacture of a medicament to treat cancer.

6. A use according to any one of claims 1 to 5 wherein, in the formula I, y is 1.

7. A use according to claim 6 wherein the compound is the propargylamine R-2-heptyl-methyl propargylamine (R-2HMP).

8. A use according to claim 6 wherein the propargylamine is selected from the group consisting of N-(1-Propyl)
N-methylpropargylamine; N-(2-Propyl) N-methylpropargylamine; N-(1-Butyl) N-methylpropargylamine; N-(1-Pentyl)
N-methylpropargylamine; N-(1-Hexyl) N-methylpropargylamine; N-(1-Heptyl) N-methylpropargylamine; N-(1-Octyl)
N-methylpropargylamine; N-(1-Nonyl) N-methylpropargylamine; N-(1-Decyl) N-methylpropargylamine; N-(1-Undecyl)
N-methylpropargylamine; N-(1-Dodecyl) N-methylpropargylamine; (R)-N-(2-Butyl) N-methylpropargylamine;(R)-N-(2-Pentyl)
N-methylpropargylamine;(R)-N-(2-Hexyl) N-methylpropargylamine; (R)-N-(2-Heptyl) N-methylpropargylamine;(R)-N-(2-Octyl)
N-methylpropargylamine; (R)-N-(2-Octyl) N-methylpropargylamine; (R)-N-(2-Decyl) N-methylpropargylamine; (R)-N-(2-Undecyl)
N-methylpropargylamine; and (R)-N-(2-Dodecyl)
N-methylpropargylamine.

9. A use according to any one of claims 1 to 5 wherein y is 0.

10. A use according to claim 9 wherein the propargylamine is R-2-heptyl-propargylamine (R-2HPA).

11. A use according to claim 9 wherein the propargylamine is selected from the group consisting of N-(1-Propyl) propargylamine; N-(2-Propyl) propargylamine; N-(1-Butyl) propargylamine; N-(1-Pentyl) propargylamine; N-(1-Hexyl) propargylamine; N-(1-Heptyl) propargylamine; N-(1-Octyl) propargylamine; N-(1-Nonyl) propargylamine; N-(1-Decyl) propargylamine; N-(1-Undecyl) propargylamine; N-(1-Dodecyl) propargylamine; (R)-N-(2-Butyl) propargylamine; (R)-N-(2-Pentyl) propargylamine; (R)-N-(2-Hexyl) propargylamine; (R)-N-(2-Heptyl) propargylamine; (R)-N-(2-Octyl) propargylamine; (R)-N-(2-Octyl) propargylamine; (R)-N-(2-Decyl) propargylamine; (R)-N-(2-Undecyl) propargylamine; and (R)-N-(2-Dodecyl) propargylamine.

12. A use according to any one of claims 1 to 6, 8, 9 or 11 wherein the propargylamine is a chiral propargylamine and is the R-enantiomer.

13. A use according to any one of claims 1-5 wherein the propargylamine is R-deprenyl.

14. A use according to any one of claims 1-5 wherein the propargylamine is R-desmethyldeprenyl.

15. A use of the propargylamine Rasagiline for the manufacture of a medicament to enhance the activity of an antineoplastic drug.

16. A use according to any one of claims 1-15 wherein the antineoplastic drug is selected from the group consisting of cytosine arabinoside, cisplatinum, cyclophospamide, adriamycin, daunomycin, and 5-fluorouracil.

17. A pharmaceutical composition for treating cancer comprising an antineoplastic drug and an effective amount of a propargylamine of the formula I as defined in claim 1.

18. A pharmaceutical composition according to claim 17 wherein the propargylamine is as defined in any of claims 6, 7, 8, 9, 10, 11, 12, 13 and 14.

19. A use for the manufacture of a medicament for treating cancer in combination with an antineoplastic drug of a propargylamine as defined in claim 1.

20. A use of a proparglyamine as defined in claim 1 for the manufacture of a medicament comprising the propargylamine and an antineoplastic drug and for treating cancer.

21. A use for the manufacture of a medicament for treating cancer in combination with a propargylamine as defined in claim 1 of an antineoplastic drug.

22. A use of any of claims 19 to 21, wherein the propargylamine is as defined in any of claims 6, 7, 8, 9, 10, 11, 12, 13 and 14.

23. A use of any of claims 19 to 22 wherein the antineoplastic drug is as defined in claim 16.

24. A use of any of claims 19 and 21, or of claim 22 or 23 when dependent on claim 19 or claim 21 wherein the medicament is for administration prior to the antineoplastic drug.

## Patentansprüche

1. Eine Verwendung eines Propargylamins zur Herstellung eines Medikaments zur Verbesserung der Aktivität einer antineoplastischen Substanz, wobei das Propargylamin folgende Allgemeinformel I aufweist: 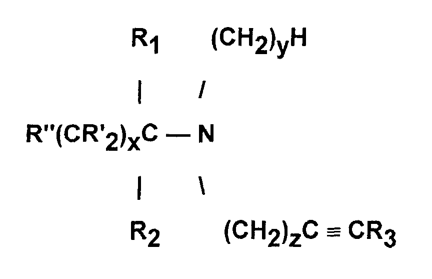 wobei
x eine ganze Zahl in dem Bereich zwischen 0 und 13 ist;
y eine ganze Zahl in dem Bereich zwischen 0 und 5 ist;
z 1 ist;
R₁, R₂ und R₃ gleich oder verschieden sind und Wasserstoff oder ein geradkettiges oder verzweigtes C₁₋₄ Alkyl darstellen; und
R' und R" gleich oder verschieden sind und Wasserstoff, Phenyl oder ein Halogen und dessen pharmazeutisch akzeptable Salze darstellen.

2. Verwendung gemäß Anspruch 1, wobei das Medikament der Erhöhung der Empfindlichkeit eines Tumors gegenüber der antineoplastischen Substanz dient.

3. Verwendung gemäß Anspruch 2, wobei der Tumor ein arzneimittelresistenter Tumor ist.

4. Verwendung gemäß Anspruch 1, wobei das Medikament dem Schutz normaler Zellen vor den zytotoxischen Wirkungen der antineoplastischen Substanz dient.

5. Verwendung (a) eines Propargylamins der Formel I gemäß Anspruch 1 und (b) einer antineoplastischen Substanz zur Herstellung eines Medikaments zur Behandlung von Krebs.

6. Verwendung gemäß einem der Ansprüche 1 bis 5, wobei y in der Formel I 1 ist.

7. Verwendung gemäß Anspruch 6, wobei die Verbindung das Propargylamin R-2-heptyl-methyl-propargylamin (R-2HMP) ist.

8. Verwendung gemäß Anspruch 6, wobei das Propargylamin aus der Gruppe ausgewählt ist, die aus N-(1-Propyl) N-methylpropargylamin; N-(2-Propyl) N-methylpropargylamin; N-(1-Butyl) N-methylpropargylamin; N-(1-Pentyl) N-methylpropargylamin; N-(1-Hexyl) N-methylpropargylamin; N-(1-Heptyl) N-methylpropargylamin; N-(1-Octyl) N-methylpropargylamin; N-(1-Nonyl) N-methylpropargylamin; N-(1-Decyl) N-methylpropargylamin; N-(1-Undecyl) N-methylpropargylamin; N-(1-Dodecyl) N-methylpropargylamin; (R)-N-(2-Butyl) N-methylpropargylamin;(R)-N-(2-Pentyl) N-methylpropargylamin; (R)-N-(2-Hexyl) N-methylpropargylamin; (R)-N-(2-Heptyl) N-methylpropargylamin; (R)-N-(2-Octyl) N-methylpropargylamin; (R)-N-(2-Octyl) N-methylpropargylamin; (R)-N-(2-Decyl) N-methylpropargylamin; (R)-N-(2-Undecyl) N-methylpropargylamin; und (R)-N-(2-Dodecyl) N-methylpropargylamin besteht.

9. Verwendung gemäß einem der Ansprüche 1 bis 5, wobei y 0 ist.

10. Verwendung gemäß Anspruch 9, wobei das Propargylamin R-2-heptyl-propargylamin (R-2HPA) ist.

11. Verwendung gemäß Anspruch 9, wobei das Propargylamin aus der Gruppe ausgewählt ist, die aus N-(1-Propyl) propargylamin; N-(2-Propyl) propargylamin; N-(1-Butyl) propargylamin; N-(1-Pentyl) propargylamin; N-(1-Hexyl) propargylamin; N-(1-Heptyl) propargylamin; N-(1-Octyl) propargylamin; N-(1-Nonyl) propargylamin; N-(1-Decyl) propargylamin; N-(1-Undecyl) propargylamin; N-(1-Dodecyl) propargylamin; (R)-N-(2-Butyl) propargylamin; (R)-N-(2-Pentyl) propargylamin; (R)-N-(2-Hexyl) propargylamin; (R)-N-(2-Heptyl) propargylamin; (R)-N-(2-Octyl) propargylamin; (R)-N-(2-Octyl) propargylamin; (R)-N-(2-Decyl) propargylamin; (R)-N-(2-Undecyl) propargylamin; und (R)-N-(2-Dodecyl) propargylamin besteht.

12. Verwendung gemäß einem der Ansprüche 1 bis 6, 8, 9 oder 11, wobei das Propargylamin ein chirales Propargylamin ist und das R-Enantiomer ist.

13. Verwendung gemäß einem der Ansprüche 1 - 5, wobei das Propargylamin R-deprenyl ist.

14. Verwendung gemäß einem der Ansprüche 1 - 5, wobei das Propargylamin R-desmethyldeprenyl ist.

15. Verwendung des Propargylamins Rasagilin zur Herstellung eines Medikaments zur Verbesserung der Aktivität einer antineoplastischen Substanz.

16. Verwendung gemäß einem der Ansprüche 1 - 15, wobei die antineoplastische Substanz aus der Gruppe ausgewählt ist, die aus Cytosin-Arabinosid, Cisplatinum, Cyclophospamid, Adriamycin, Daunomycin und 5-Fluorouracil besteht.

17. Eine pharmazeutische Zusammensetzung zur Behandlung von Krebs, die eine antineoplastische Substanz und eine wirksame Menge an Propargylamin der Formel I gemäß Anspruch 1 umfasst.

18. Pharmazeutische Zusammensetzung gemäß Anspruch 17, wobei Propargylamin wie gemäß einem der Ansprüche 6, 7, 8, 9, 10, 11, 12, 13 und 14 definiert ist.

19. Verwendung zur Herstellung eines Medikaments zur Behandlung von Krebs in Kombination mit einer antineoplastischen Substanz eines Propargylamins gemäß Anspruch 1.

20. Verwendung eines Propargylamins gemäß Anspruch 1 zur Herstellung eines Medikaments, das das Propargylamin und eine antineoplastische Substanz umfasst und zur Behandlung von Krebs dient.

21. Verwendung zur Herstellung eines Medikaments zur Behandlung von Krebs in Kombination mit einem Propargylamin gemäß Anspruch 1 einer antineoplastischen Substanz.

22. Verwendung gemäß einem der Ansprüche 19 bis 21, wobei das Propargylamin wie gemäß einem der Ansprüche 6, 7, 8, 9, 10, 11, 12, 13 und 14 definiert ist.

23. Verwendung gemäß einem der Ansprüche 19 bis 22, wobei die antineoplastische Substanz wie gemäß Anspruch 16 ist.

24. Verwendung gemäß einem der Ansprüche 19 und 21 oder gemäß Anspruch 22 oder 23, wenn von Anspruch 19 oder Anspruch 21 abhängig, wobei das Medikament zur Verabreichung vor der antineoplastischen Substanz ist.

## Revendications

1. Une utilisation d'une amine propargylique pour la fabrication d'un médicament pour renforcer l'activité d'une drogue antinéoplastique dans laquelle l'amine propargylique est de la formule générale I 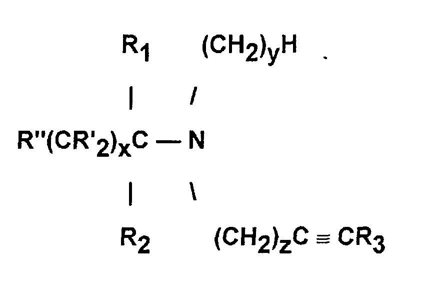 dans laquelle
x est un nombre entier allant de 0 à 13 ;
y est un nombre entier allant de 0 à 5 ;
z est 1 ;
R₁, R₂ et R₃ sont les mêmes ou sont différents et représentent l'hydrogène ou un alkyle C₁₋₄ à chaîne droite ou ramifiée ; et
R' et R" sont les mêmes ou sont différents et représentent de l'hydrogène, le phényle ou un halogène et des sels de ceux-ci acceptables du point de vue pharmaceutique.

2. Une utilisation selon la revendication 1 dans laquelle le médicament est destiné à accroître la sensibilité d'une tumeur à la drogue antinéoplastique.

3. Une utilisation selon la revendication 2 dans laquelle la tumeur est une tumeur résistante à la drogue.

4. Une utilisation selon la revendication 1 dans laquelle le médicament est destiné à protéger des cellules normales des effets cytotoxiques de la drogue antinéoplastique.

5. Une utilisation de (a) une amine propargylique de la formule I telle que définie dans la revendication 1 et (b) une drogue antinéoplastique pour la fabrication d'un médicament pour traiter le cancer.

6. Une utilisation selon n'importe laquelle des revendications 1 à 5 dans laquelle, dans la formule I, y est 1.

7. Une utilisation selon la revendication 6 dans laquelle le composé est l'amine propargylique R-2-heptyl-méthyl propargylamine (R-2HMP).

8. Une utilisation selon la revendication 6 dans laquelle l'amine propargylique est sélectionnée dans le groupe consistant en N-(1-Propyl) N-méthylpropargylamine ; N-(2-Propyl) N-méthylpropargylamine ; N-(1-Butyl) N-méthylpropargylamine ; N-(1-Pentyl) N-méthylpropargylamine ; N-(1-Héxyl) N-méthylpropargylamine ; N-(1-Heptyl) N-méthylpropargylamine ; N-(1-Octyl) N-méthylpropargylamine ; N-(1-Nonyl) N-méthylpropargylamine ; N-(1-Décyl) N-méthylpropargylamine ; N-(1-Undécyl) N-méthylpropargylamine ; N-(1-Dodécyl) N-méthylpropargylamine ; (R)-N-(2-Butyl) N-méthylpropargylamine ; (R)-N-(2-Pentyl) N-méthylpropargylamine ; (R)-N-(2-Héxyl) N-méthylpropargylamine ; (R)-N-(2-Heptyl) N-méthylpropargylamine ; (R)-N-(2-Octyl) N-méthylpropargylamine ; (R)-N-(2-Octyl) N-méthylpropargylamine ; (R)-N-(2-Décyl) N-méthylpropargylamine ; (R)-N-(2-Undécyl) N-méthylpropargylamine ; et (R)-N-(2-Dodécyl) N-méthylpropargylamine.

9. Une utilisation selon n'importe laquelle des revendications 1 à 5 dans laquelle y est 0.

10. Une utilisation selon la revendication 9 dans laquelle l'amine propargylique est la R-2-heptyl-propargylamine (R-2HPA).

11. Une utilisation selon la revendication 9 dans laquelle l'amine propargylique est sélectionnée dans le groupe consistent en N-(1-Propyl) propargylamine ; N-(2-Propyl) propargylamine ; N-(1-Butyl) propargylamine ; N-(1-Pentyl) propargylamine ; N-(1-Héxyl) propargylamine ; N-(1-Heptyl) propargylamine ; N-(1-Octyl) propargylamine ; N-(1-Nonyl) propargylamine ; N-(1-Décyl) propargylamine ; N-(1-Undécyl) propargylamine ; N-(1-Dodécyl) propargylamine ; (R)-N-(2-Butyl) propargylamine ; (R)-N-(Z-Pentyl) propargylamine ; (R)-N-(2-Héxyl) propargylamine ; (R)-N-(2-Heptyl) propargylamine ; (R)-N-(2-Octyl) propargylamine ; (R)-N-(2-Octyl) propargylamine ; (R)-N-(2-Décyl) propargylamine ; (R)-N-(Z-Undécyl) propargylamine ; et (R)-N-(2-Dodécyl) propargylamine.

12. Une utilisation selon n'importe laquelle des revendications 1 à 6, 8, 9 ou 11 dans laquelle famine propargylique est une amine propargylique chirale et est l'énantiomère R.

13. Une utilisation selon n'importe laquelle des revendications 1 à 5 dans laquelle famine propargylique est le déprényl R.

14. Une utilisation selon n'importe laquelle des revendications 1 à 5 dans laquelle famine propargylique est le desméthyldéprényl R.

15. Une utilisation de famine propargylique Rasagiline pour la fabrication d'un médicament pour renforcer l'activité dune drogue antinéoplastique.

16. Une utilisation selon n'importe laquelle des revendications 1 à 15 dans laquelle la drogue antinéoplastique est selectionnée dans le groupe consistant en cytosine arabinoside, cisplatinum, cyclophospamide, adriamycine, daunomycine et 5-fluorouracil.

17. Une composition pharmaceutique pour le traitement du cancer comprenant une drogue antinéoplastique et une quantité efficace dune amine propargylique de la formule I telle que définie dans la revendication 1.

18. Une composition pharmaceutique selon la revendication 17 dans laquelle famine propargylique est telle que définie dans n'importe lesquelles des revendications 6, 7, 8, 9, 10, 11, 12, 13 et 14.

19. Une utilisation, pour la fabrication d'un médicament pour le traitement du cancer en combinaison avec une drogue antinéoplastique, dune amine propargylique telle que définie dans la revendication 1.

20. Une utilisation dune amine propargylique telle que définie dans la revendication 1 pour la fabrication d'un médicament comprenant famine propargylique et une drogue antinéoplastique et pour le traitement du cancer.

21. Une utilisation, pour la fabrication d'un médicament pour le traitement du cancer en combinaison avec une amine propargylique telle que définie dans la revendication 1, dune drogue antinéoplastique.

22. Une utilisation de n'importe lesquelles des revendications 19 à 21, dans laquelle famine propargylique est telle que définie dans n'importe lesquelles des revendications 6, 7, 8, 9, 10, 11, 12, 13 et 14.

23. Une utilisation de n'importe lesquelles des revendications 19 à 22 dans laquelle la drogue antinéoplastique est telle que définie dans la revendication 16.

24. Une utilisation de n'importe lesquelles des revendications 19 à 21, ou des revendications 22 ou 23 lorsqu'elles dépendent de la revendication 19 ou de la revendication 21, dans laquelle le médicament est destiné à être administré avant la drogue antinéoplastique.
